(19) Europäisches Patentamt
European Patent Office
Office européen des brevets

(11) EP 1 437 342 A2

(12) EUROPÄISCHE PATENTANMELDUNG

(43) Veröffentlichungstag:
**14.07.2004 Patentblatt 2004/29**

(21) Anmeldenummer: **03029976.2**

(22) Anmeldetag: **30.12.2003**

(51) Int Cl.[7]: **C07C 211/15**, C07C 211/29, C07D 213/38, C07D 295/06, C07C 211/27, C07D 207/10, C07B 39/00

(84) Benannte Vertragsstaaten:
**AT BE BG CH CY CZ DE DK EE ES FI FR GB GR HU IE IT LI LU MC NL PT RO SE SI SK TR**
Benannte Erstreckungsstaaten:
**AL LT LV MK**

(30) Priorität: **07.01.2003 DE 10300112**

(71) Anmelder: **Bayer Chemicals AG**
**51368 Leverkusen (DE)**

(72) Erfinder:
- **Ebenbeck, Wolfgang, Dr.**
  **51373 Leverkusen (DE)**
- **Hilgers, Petra, Dr.**
  **51503 Rösrath (DE)**
- **Marhold, Albrecht, Dr.**
  **51373 Leverkusen (DE)**
- **Barten, Jan Alexander, Dr.**
  **28359 Bremen (DE)**
- **Kolomeitsev, Alexander, Dr.**
  **28205 Bremen (DE)**
- **Röschenthaler, Gerd-Volker, Prof. Dr.**
  **28357 Bremen (DE)**

(54) **Fluorierungsreagenzien und Verfahren zu deren Herstellung**

(57) Die vorliegende Erfindung betrifft α,α-Difluoramine, Fluorierungsreagenzien enthaltend α,α-Difluoramine sowie Verfahren zur Herstellung von α,α-Difluoraminen und Fluorierungsreagenzien enthaltend α,α-Difluoramine.

EP 1 437 342 A2

## Beschreibung

Die vorliegende Erfindung betrifft α,α-Difluoramine, Fluorierungsreagenzien enthaltend α,α-Difluoramine sowie Verfahren zur Herstellung von α,α-Difluoraminen und Fluorierungsreagenzien enthaltend α,α-Difluoramine.

Als Reagenzien zur Fluorierung von Alkoholen oder Carbonylverbindungen wie insbesondere Ketonen, Carbonsäuren und Aldehyden sind beispielsweise Schwefeltetrafluorid, Diethylaminoschwefeltrifluorid (DAST) und Bis-(methoxyethyl)-aminoschwefeltrifluorid (Methoxy-DAST) bekannt (siehe auch US 3,976,691, EP-A 90 448 und EP-A 905 109). Nachteilig am industriellen Einsatz von Schwefeltetrafluorid ist dessen extrem hohe Toxizität und die Notwendigkeit von umfangreichen Sicherheitsmaßnahmen, die genannten Aminoschwefeltrifluoride sind darüberhinaus stoßempfindlich (*J. Fluorine Chem.* **1989**, *42*, 137) und unterliegen aufgrund ihrer Explosivität strengen gesetzlichen Auflagen.

Ein weiteres Reagenz zur Fluorierung von sekundären Alkoholen und Carbonsäuren ist N,N-Dimethyl-1,1-difluorbenzylamin, das durch Umsetzung von N,N-Dimethylbenzamid mit Schwefeltetrafluorid bei 150°C erhältlich ist [*J. Fluorine Chem.* **1983**, *23*, 219-228]. Das Reagenz ist jedoch in seiner Anwendungsbreite beschränkt und liefert nur mittlere Ausbeuten.

Weiterhin ist als Fluorierungsmittel für Alkohole 2-Chlor-1,1,2-trifluortriethylamin, das sogenannte Yarovenko-Reagenz bekannt (*Org. React.* **1974**, *21*, 158). Das Reagenz ist jedoch nicht lagerbeständig und nur unter großem Aufwand herstellbar.

Ein ähnliches Reagenz, das unter dem Namen Ishikawa-Reagenz geläufig ist, besteht aus einer Mischung von Hexafluorpropyl-dialkylamin und Pentafluoralkenyl-dialkylamin, weist jedoch die gleichen oben genannten Nachteile auf.

Aus EP-A 895 991 sind Difluormethylen-α,α-diazoverbindungen bekannt, die zur Fluorierung von Hydroxy- und Carboxylfunktionen eingesetzt werden können. Aufgrund ihrer hohen Empfindlichkeit gegen Luft und Feuchtigkeit sind sie für den technischen Einsatz jedoch nur bedingt geeignet.

Es bestand daher das Bedürfnis, Fluorierungsreagenzien bereitzustellen, die sowohl effizient aus einfach verfügbaren Edukten hergestellt werden können, lagerstabil sind und die Hydroxy- und Ketofunktionen in guten Ausbeuten fluorieren können.

Es wurden nun Verbindungen der Formel (I) gefunden,

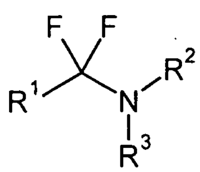

(I)

in der

| | |
|---|---|
| $R^1$ | für Wasserstoff, $C_1$-$C_{12}$-Alkyl, $[(C_2\text{-}C_{12}\text{-Alkylen})\text{-O}]_n(C_1\text{-}C_{12}\text{-alkyl})]$ mit n = 1 bis 5, $C_4$-$C_{15}$-Arylalkyl oder $C_3$-$C_{14}$-Heteroaryl steht |
| $R^2$ und $R^3$ | jeweils unabhängig voneinander für $C_4$-$C_{15}$-Arylalkyl oder $C_1$-$C_{12}$-Alkyl stehen oder gemeinsam Teil eines cyclischen Restes mit insgesamt 3 bis 12 Kohlenstoffatomen sind oder |
| $R^1$ und $R^2$ und/oder $R^3$ | gemeinsam Teil eines cyclischen Restes mit insgesamt 3 bis 12 Kohlenstoffatomen sind |

wobei 1,1-Difluormethyl-N,N-dimethylamin, 1,1-Difluormethyl-N,N-diethylamin, 1,1-Difluormethyl-N,N-diisopropylamin und 1,1-Difluor-N,N-2-trimethyl-1-propanamin ausgenommen sind.

Im Rahmen der Erfindung können alle aufgeführten, allgemeinen oder in Vorzugsbereichen genannten Restedefinitionen und Parameter untereinander, also auch zwischen den jeweiligen Bereichen und Vorzugsbereichen in beliebiger Weise kombiniert werden.

Es sei angemerkt, dass die aus Vereinfachungsgründen gewählte Darstellung der Formel (I) auch die oftmals in der Literatur benutzte nachstehende Darstellung umfasst.

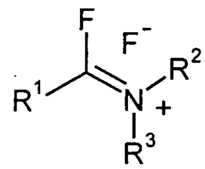

Das Gleiche gilt im Rahmen der Erfindung analog für alle anderen Darstellungen und Nomenklaturen von α,α-Dihalogenoamin-Funktionalitäten.

**Alkyl** beziehungsweise **Alkylen** beziehungsweise **Alkoxy** steht jeweils unabhängig für einen geradkettigen, cyclischen, verzweigten oder unverzweigten Alkyl beziehungsweise Alkylen beziehungsweise Alkoxy-Rest. Gleiches gilt für den nichtaromatischen Teil eines Arylalkyl-Restes.

$C_1$-$C_4$-Alkyl steht beispielsweise für Methyl, Ethyl, n-Propyl, iso-Propyl, n-Butyl, sec.-Butyl und tert.-Butyl, $C_1$-$C_8$-Alkyl darüber hinaus beispielsweise für n-Pentyl, 1-Methylbutyl, 2-Methylbutyl, 3-Methylbutyl, neo-Pentyl, 1-Ethylpropyl, cyclo-Hexyl, cyclo-Pentyl, n-Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, n-Heptyl und n-Octyl, $C_1$-$C_{12}$-Alkyl weiter darüber hinaus beispielsweise für Adamantyl, die isomeren Menthyle, n-Nonyl, n-Decyl und n-Dodecyl.

$C_1$-$C_4$-Alkoxy steht beispielsweise für Methoxy, Ethoxy, n-Propoxy, iso-Propoxy, n-Butoxy, sec.-Butoxy und tert.-Butoxy, $C_1$-$C_8$-Alkoxy darüberhinaus für n-Pentoxy, 1-Methylbutoxy, 2-Methylbutoxy, 3-Methylbutoxy, neo-Pentoxy, 1-Ethylpropoxy, cyclo-Hexoxy, cyclo-Pentoxy, n-Hexoxy und n-Octoxy, $C_1$-$C_{12}$-Alkoxy weiter darüber hinaus beispielsweise für Adamantoxy, die isomeren Menthoxy-Reste, n-Decoxy und n-Dodecoxy.

$C_2$-$C_{12}$-Alkylen steht beispielsweise für 1,2-Ethylen, 1,3-Propylen, 1,4-Butylen, 1,2-cyclo-Hexoxylen und 1,2-cyclo-Pentylen.

**Heteroaryl** steht jeweils unabhängig für einen heteroaromatischen Rest mit 3 bis 14 Gerüstkohlenstoffatomen, wobei weiterhin keines, ein, zwei oder drei Gerüstkohlenatome pro Zyklus, im gesamten Molekül mindestens jedoch ein Gerüstatom ausgewählt ist aus der Gruppe Stickstoff, Schwefel oder Sauerstoff.

Beispiele für heteroaromatische Reste sind Pyridinyl, Oxazolyl, Benzofuranyl, Dibenzofuranyl oder Chinolinyl.

Weiterhin kann der heteroaromatische Rest mit bis zu fünf gleichen oder verschiedenen Substituenten pro Zyklus substituiert sein, die ausgewählt sind aus der Gruppe Chlor, Fluor, $C_1$-$C_{12}$-Alkyl, $C_1$-$C_{12}$-Fluoralkyl, $C_1$-$C_{12}$-Fluoralkoxy, $C_1$-$C_{12}$-Fluoralkylthio, $C_1$-$C_{12}$-Alkoxy, Di($C_1$-$C_8$-alkyl)amino und Tri($C_1$-$C_8$-alkyl)siloxyl.

**Aryl** steht jeweils unabhängig für einen Heteroaryl-Rest gemäß obiger Definition oder für einen carbocyclischen aromatischen Rest.

Beispiele für carbocyclische aromatische Reste mit 6 bis 14 Gerüstkohlenstoffatomen sind Phenyl, Naphtyl, Phenanthrenyl, Anthracenyl oder Fluorenyl.

Weiterhin kann der carbocyclische aromatische Rest wie für die heteroaromatischen Reste oben beschrieben substituiert sein.

**Arylalkyl** steht jeweils unabhängig für einen geradkettigen, cyclischen, verzweigten oder unverzweigten Alkyl-Rest nach vorstehender Definition, der einfach, mehrfach oder vollständig durch Aryl-Reste gemäß vorstehender Definition substituiert ist.

Im Folgenden werden die bevorzugten Substitutionsmuster für Verbindungen der Formel (I) definiert:

$R^1$ steht bevorzugt für Wasserstoff, $C_1$-$C_{12}$-Alkyl, oder $C_3$-$C_5$-Heteroaryl, besonders bevorzugt für Wasserstoff oder $C_1$-$C_8$-Alkyl und ganz besonders bevorzugt für Wasserstoff oder $C_1$-$C_4$-Alkyl

$R^2$ und $R^3$ stehen bevorzugt jeweils unabhängig voneinander für $C_1$-$C_8$-Alkyl oder $NR^2R^3$ als Ganzes für N-Morpholinyl, N-Methyl-1,4-Piperazin-N-yl, und besonders bevorzugt jeweils identisch für Methyl, Ethyl oder iso-Propyl.

Ebenfalls bevorzugt steht die Formel (I) als Ganzes für 2,2-Difluorpyrrolidin, 2,2-Difluorpiperidin, [2.2.2]-2,2,5,5-Tetrafluor-1,4-diazabicyclooctan oder [2.2.2]-2,2,6,6-Tetrafluor-1,4-diazabicyclooctan wobei die genannten Reste gegebenenfalls einfach oder mehrfach durch $C_1$-$C_4$-Alkyl substituiert sind.

Als Verbindungen der Formel (I) seien genannt:

1,1-Difluor-N,N-2,2-tetramethyl-1-propanamin, N,N-Diethyl-α,α-difluor-2,2-dimethyl-1-propanamin, N-(1,1-Difluormethyl)-morpholin, N,N-Diethyl-α,α-difluor-3-pyridylmethanamin, N,N-Diethyl-α,α-difluor-2-pyridylmethanamin und 2,2-Difluor-1,3,3-trimethylpyrrolidin.

Überraschenderweise wurde gefunden, dass Verbindungen der Formel (Ia), unter der sich die erfindungsgemäßen Verbindungen der Formel (I) subsumieren lassen, als Fluorierungsreagenz noch effizienter wirken, wenn sie in Gegenwart eines tertiären aprotischen Amins und/oder einer N-heteroaromatischen Verbindung und in Gegenwart von Fluorwasserstoff eingesetzt werden.

Daher sind von der Erfindung auch Mischungen umfasst, enthaltend

- Verbindungen der Formel (Ia)

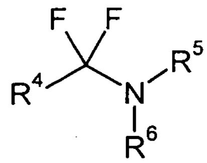

(Ia)

in der

| | |
|---|---|
| $R^4$ | für Wasserstoff, $C_1$-$C_{12}$-Alkyl, $[(C_2$-$C_{12}$-Alkylen)-O$]_n(C_1$-$C_{12}$-alkyl)] mit n = 1 bis 5, $C_3$-$C_{14}$-Aryl oder $NR^7R^8$ steht, wobei $R^7$ und $R^8$ jeweils unabhängig voneinander für $C_1$-$C_8$-Alkyl stehen oder $NR^7R^8$ als Ganzes für einen 4- bis 7-gliedrigen cyclischen Rest mit insgesamt 3 bis 12 Kohlenstoffatomen steht und |
| $R^5$ und $R^6$ | jeweils unabhängig voneinander für $C_1$-$C_{12}$-Alkyl stehen oder gemeinsam Teil eines cyclischen Restes mit insgesamt 4 bis 12 Kohlenstoffatomen sind oder |
| $R^4$ und $R^5$ und/oder R6 | gemeinsam Teil eines cyclischen Restes mit insgesamt 4 bis 12 Kohlenstoffatomen sind, |

- zumindest ein, bevorzugt genau ein aprotisches, tertiäres Amin, das in α-Stellung zum Stickstoff keine Fluoratome enthält und/oder zumindest eine, bevorzugt genau eine N-heteroaromatische Verbindung und

- Fluorwasserstoff.

Aprotisch bedeutet in diesem Zusammenhang, dass das tertiäre Amin, das auch ein Molekül mit mehreren tertiären Aminogruppen sein kann, keine Wasserstoffatome trägt die bezogen auf eine wässrige Vergleichsskala bei 25°C einen pKs-Wert von weniger als 20 aufweisen.

Es sei angemerkt, dass unter den oben aus Vereinfachungsgründen gewählten Begrifflichkeiten auch die entsprechenden tertiären Ammoniumfluoride. und N-heteroaryliumfluoride und die entsprechenden Polyfluoride umfasst sind, die bei der Reaktion mit Fluorwasserstoff auftreten.

Bevorzugte Verbindungen der Formel (Ia) sind solche der Formel (I) mit der vorstehend genannten Bedeutung und solche der Formeln (Ib), (Ic), (Id) und (Ie)

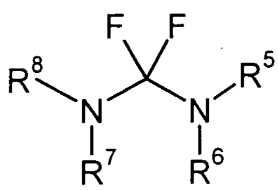

(Ib)

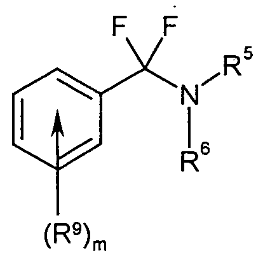

(Ic)

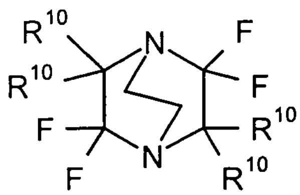
(Id)

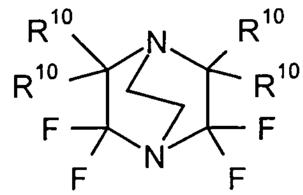
(Ie)

in denen $R^5$, $R^6$, $R^7$ und $R^8$ die vorstehend genannte Bedeutung besitzen, m für 0, 1, 2, 3 oder 4 steht und $R^9$ für Reste steht, die ausgewählt sind aus der Gruppe Chlor, Fluor, $C_1$-$C_{12}$-Alkyl, $C_1$-$C_{12}$-Fluoralkyl, $C_1$-$C_{12}$-Fluoralkoxy, $C_1$-$C_{12}$-Fluoralkylthio, $C_1$-$C_{12}$-Alkoxy und Di($C_1$-$C_8$-alkyl)-amino und $R^{10}$ jeweils unabhängig für Wasserstoff oder $C_1$-$C_{12}$-Alkyl steht.

Als Verbindung der Formel (Ib) sei insbesondere 2,2'-Difluor-1,3-dimethylimidazolidin genannt, als Verbindungen der Formel (Ic) insbesondere N,N-Diethyl-α,α-difluor-phenyl-methanamin, N,N-Dimethyl-α,α-difluor-phenyl-methanamin, N,N-Di-isopropyl-α,α-difluor-phenyl-methanamin und Diethyl-α,α-difluor-(4-chlorphenyl)-methanamin, als Verbindung der Formel (Id) [2.2.2]-2,2,5,5-Tetrafluor-3,3,6,6-tetramethyl-1,4-diazabicyclooctan, als Verbindung der Formel (Ie) [2.2.2]-2,2,6,6-Tetrafluor-3,3,5,6-tetramethyl-1,4-diazabicyclooctan.

Bevorzugte aprotische tertiäre Amine sind solche der Formeln (IVa) und (IVb)

$$NR^{11}R^{12}R^{13} \qquad \text{(IVa)}$$

$$(R^{14})_2N\text{-}L\text{-}N(R^{14})_2 \qquad \text{(IVb)}$$

in denen $R^{11}$, $R^{12}$ und $R^{13}$ jeweils unabhängig voneinander für $C_1$-$C_{12}$-Alkyl oder [($C_2$-$C_{12}$-Alkylen)-O]$_n$($C_1$-$C_{12}$-alkyl)] mit n = 1 bis 5 stehen oder zwei oder drei der Reste $R^{10}$, $R^{11}$ und/oder $R^{12}$ mit dem Stickstoffatom einen mono- bzw. bi-cyclischen Rest mit insgesamt 3 bis 12 bzw. 5 bis 15 Kohlenstoffatomen bilden, L für $C_2$-$C_6$-Alkylen steht und die Reste $R^{14}$ jeweils unabhängig voneinander für $C_1$-$C_8$-Alkyl stehen oder zwei Reste zusammen für $C_2$-$C_6$-Alkylen stehen.

In Formel (IVa) stehen bevorzugt $R^{11}$, $R^{12}$ und $R^{13}$ jeweils unabhängig voneinander für $C_1$-$C_{12}$-Alkyl, besonders bevorzugt jeweils identisch für $C_1$-$C_8$-Alkyl.

Besonders bevorzugte aprotische tertiäre Amine sind Triethylamin, Tetramethylethylendiamin und [2.2.2]-1,4-Diazabicyclooctan.

Bevorzugte N-heterocyclische Verbindungen sind gegebenenfalls substituierte Pyridine und Chinoline, wobei Pyridin besonders bevorzugt ist.

Im Rahmen der Erfindung ist der Einsatz von Triethylamin ganz besonders bevorzugt.

Das molare Verhältnis von aprotischem tertiärem Amin oder N-heteroaromatischer Verbindung zu Verbindungen der Formel (Ia) beträgt beispielsweise und bevorzugt 0,1:1 bis 20:1, bevorzugt 1:1 bis 10:1 und besonders bevorzugt 1:1 1 bis 5:1.

Das molare Verhältnis von Fluorwasserstoff zu aprotischem tertiärem Amin oder N-heteroaromatischer Verbindung beträgt beispielsweise und bevorzugt 0,2:1 bis 10:1, pro Stickstoffatom.

Die erfindungsgemäßen Mischungen enthaltend Verbindungen der Formel (Ia), zumindest ein aprotisches tertiäres Amin oder N-heteroaromatische Verbindung und Fluorwasserstoff sind beispielsweise durch Mischung der Verbindungen der Formel (Ia) mit aprotischem tertiären Amin oder N-heteroaromatischer Verbindung und Fluorwasserstoff erhältlich oder durch Mischung der Verbindungen der Formel (Ia) mit Mischungen aus aprotischem tertiärem Amin oder N-heteroaromatischer Verbindung und Fluorwasserstoff, die in verschiedenen Zusammensetzungen wie z. B. ($NEt_3$ x 3 HF) oder (Pyridin x 9HF) auch kommerziell erhältlich sind.

Die Verbindungen der Formel (I) lassen sich in besonders vorteilhafter Weise dadurch herstellen, dass Verbindungen der Formel (V)

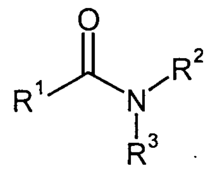

(V)

in der $R^1$, $R^2$ und $R^3$ die obenstehend angegebene Bedeutung einschließlich der genannten Vorzugsbereiche besitzen

- in einem Schritt a) mit Halogenierungsmittel in Verbindungen der Formel (VI) überführt werden

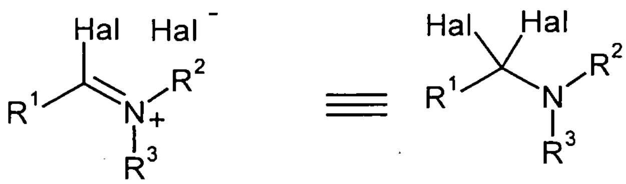

(VI)

in der Hal jeweils unabhängig für Chlor oder Brom steht und

- in einem Schritt b) die Verbindungen der Formel (VI) mit ionischem Fluorid in Verbindungen der Formel (I) überführt werden

Bevorzugte Halogenierungsmittel für Schritt a) sind Phosphorpentachlorid, Phosphorpentabromid, Thionylchlorid, Thionylbromid, Phosgen und/oder Oxalsäurechlorid, wobei Phosphorpentachlorid, Thionylchlorid, Phosgen und/oder Oxalsäurechlorid noch weiter bevorzugt sind.

Das molare Verhältnis von Halogenierungsmittel zu Verbindung der Formel (V) beträgt beispielsweise und bevorzugt 0,9:1 bis 10:1, bevorzugt 1:1 bis 2:1 und besonders bevorzugt 1,02:1 bis 1,5:1.

Als Lösungsmittel für Schritt a) können aliphatische, alicyclische oder aromatische, gegebenenfalls halogenierte Kohlenwasserstoffe, wie beispielsweise Benzin, Benzol, Toluol, Xylol, Chlorbenzol, die isomeren Dichlorbenzole, Petrolether, Hexan, Cyclohexan, Dichlormethan, Chloroform und/oder Ether, wie Diethylether, Diisopropylether, Dioxan, Tetrahydrofuran oder Ethylenglykoldimethyl- oder -diethylether eingesetzt werden.

Die Reaktionstemperatur in Schritt a) kann beispielsweise -20°C bis zum Siedepunkt des eingesetzten Lösungsmittel bei Reaktionsdruck betragen maximal jedoch 150°C, bevorzugt -10°C bis zum Siedepunkt des eingesetzten Lösungsmittel bei Reaktionsdruck, maximal jedoch 50°C.

Der Reaktionsdruck in Schritt a) kann beispielsweise 0,8 bis 20 bar betragen, bevorzugt sind 0,9 bis 3 bar, wobei Umgebungsdruck noch weiter bevorzugt ist.

Die Aufarbeitung nach der Reaktion kann z.B. durch Abdestillieren aller flüchtigen Bestandteile und Trocknen des Rückstandes im Hochvakuum erfolgen.

Die gemäß Schritt a) erhältlichen Verbindungen der Formel (VI) sind als für das genannte Herstellungsverfahren unverzichtbare Zwischenprodukte ebenfalls von der Erfindung umfasst.

Als Verbindungen der Formel (VI) seien genannt:

1,1-Dichlormethyl-N,N-dimethylamin, 1,1-Dichlormethyl-N,N-diethylamin, 1,1-Dichlormethyl-N,N-diisopropylamin, 1, 1-Dichlor-N,N-2-trimethyl- 1-propanamin, 1 , 1-Dichlor-N,N-2,2-tetramethyl-1-propanamin, N,N-Diethyl-α, α-dichlor-2,2-dimethyl-1-propanamin, N-(1,1-Dichlormethyl)-morpholin, N,N-Diethyl-α,α-dichlor-3-pyridylmethanamin, N,N-Diethyl-α,α-dichlor-2-pyridylmethanamin und 2,2-Dichlor-1,3,3-trimethylpyrrolidin.

Gemäß Schritt b) werden die Verbindungen der Formel (VI) mit ionischem Fluorid umgesetzt.

Ionische Fluoride sind beispielsweise quartäre Ammonium- oder Phosphoniumfluoride sowie Alkalimetallfluoride oder Mischungen der genannten Verbindungen.

Beispiele für Ammonium- oder Phosphoniumfluoride sind solche der Formel (VII),

$$(\text{Kation}^{+})(\text{F}^{-}) \quad \text{(VII)}$$

in der

(Kation$^+$) für Kationen der Formel (VIII) steht

$$[Pnyc(C_1\text{-}C_{12}\text{-Alkyl})_q(C_6\text{-}C_{15}\text{-Arylalkyl})_r(C_3\text{-}C_{14}\text{-Aryl})_s(\{(C_2\text{-}C_8\text{-Alkylen})\text{-}O]_v\text{-}(C_1\text{-}C_8\text{-Alkyl})\}_t)]+ \qquad \text{(VIII)}$$

wobei
Pnyc für Stickstoff oder Phosphor steht und in der (q+r+s+t) = 4 ist.

Bevorzugt ist jedoch der Einsatz von Alkalimetallfluoriden oder Mischungen von Alkalimetallfluoriden, wobei Natrium-, Kalium- und Cäsiumfluorid besonders bevorzugt und Natriumfluorid ganz besonders bevorzugt ist.

Das molare Verhältnis von ionischem Fluorid zu eingesetzter Verbindung der Formel (VI) kann beispielsweise 0,7 bis 5 betragen, vorzugsweise 0,9 bis 2 und besonders bevorzugt 1,1 bis 1,7. Die einsetzbare Menge an ionischem Fluorid ist nach oben lediglich durch wirtschaftliche Überlegungen begrenzt.

Vorzugsweise wird Schritt b) in einem organischen Lösungsmittel durchgeführt. Als organische Lösungsmittel sind beispielsweise geeignet: Nitrile, wie Acetonitril, Propionitril, Benzonitril, Benzylnitril, oder Butyronitril; Amide wie N,N-Dimethylformamid, N,N-Dimethylacetamid, N-Methyl-formanilid, N-Methyl-pyrrolidon und Dimethylimidazolidinon sowie die als Ausgangsverbindungen für die Herstellung der Verbindungen der Formel (VI) eingesetzten Amide oder Sulfoxide, wie Dimethylsulfoxid, Sulfone wie Tetramethylensulfon, Polyether wie 1,4-Dioxan, Ethylenglykoldimethylether, Ethylenglykoldiethylether, Diethylenglykoldimethylether, Diethylenglykoldiethylether, Benzotrifluoride oder Gemische solcher organischen Lösungsmittel.

Vorzugsweise beträgt der Wassergehalt des Lösungsmittels im erfindungsgemäßen Verfahren maximal 0,2 Gew.-%, bevorzugt maximal 0,05 Gew.-%. Vorzugsweise wird ein solcher Wassergehalt durch Andestillieren oder Trocknung in an sich bekannter Weise erreicht. Bei Einsatz von Alkalimetallfluoriden wird besonders bevorzugt das Lösungsmittel gleichzeitig in Gegenwart des eingesetzten Alkalimetallfluorids getrocknet bzw. andestilliert.

Die Reaktionstemperatur in Schritt b) kann beispielsweise 60°C bis zum Siedepunkt des eingesetzten Lösungsmittel bei Reaktionsdruck betragen maximal jedoch 180°C, bevorzugt 110°C bis zum Siedepunkt des eingesetzten Lösungsmittel bei Reaktionsdruck maximal jedoch 150°C betragen.

Der Reaktionsdruck kann beispielsweise 0,8 bis 30 bar betragen, bevorzugt sind 1 bis 2 bar.

Gegebenenfalls kann die Reaktivität der ionischen Fluoride durch Zusätze modifiziert werden. Geeignete Zusätze sind beispielsweise Phasentransferkatalysatoren.

Als Phasentransferkatalysatoren sind beispielsweise Kronenether, wie 18-Krone-6, 12-Krone-4, Dibenzo-18-Krone-6 oder Dibenzo-12-Krone-4, Kryptanden wie Kryptand[2.2.2] oder Podanden wie Polyglykolether oder solche der Formel (IX) geeignet,

$$(\text{Kation}^+)(\text{Anion}^-) \qquad \text{(IX)}$$

in der

(Kation$^+$) vorstehend genannte Bedeutung und Vorzugsbereiche besitzt und

(Anion$^-$) für das Anion einer organischen oder anorganischen Säure steht.

Auf beschriebene Weise werden die Verbindungen der Formel (I) nach Aufarbeitung die beispielsweise wie für Verbindungen der Formel (VI) ausgeführt werden kann, in hoher Ausbeute und Reinheit erhalten.

Für die Herstellung von Verbindungen der Formel (Ia) und insbesondere für die Herstellung der erfindungsgemäßen Mischungen hat es sich besonders bewährt die Verbindungen der Formel (VI)

- in einem Schritt b*)

in Gegenwart von Fluorwasserstoff umzusetzen und gegebenenfalls die auf diese Weise erhaltene Reaktionsmischung mit aprotischem tertiärem Amin das in α-Stellung zum Stickstoff keine Fluoratome enthält und/oder N-heteroaromatischer Verbindung umzusetzen.

Der Ausdruck „in Gegenwart von Fluorwasserstoff" schließt die Möglichkeit ein, Mischungen von Fluorwasserstoff mit aprotischen tertiären Aminen, die in α-Stellung zum Stickstoff keine Fluoratome enthalten und/oder N-heteroaromatische Verbindungen einzusetzen, in denen der Fluorwasserstoff molar im Überschuss vorhanden ist. Solche Mischungen sind beispielsweise die oben erwähnten Mischungen ($NEt_3$ x 3 HF) und (Pyridin x 9HF).

Bevorzugt wird Schritt b*) jedoch so durchgeführt, dass die erfindungsgemäßen Mischungen so hergestellt werden, dass Verbindungen der Formel (VI) mit so viel Fluorwasserstoff umgesetzt werden und die so erhaltene Reaktionsmischung mit so viel aprotischem, tertiären Amin das in α-Stellung zum Stickstoff keine Fluoratome enthält und/oder N-heteroaromatischer Verbindung umgesetzt wird, dass die für die erfindungsgemäßen Mischungen vorstehend genannten Mischungsverhältnisse eingehalten werden. Die genannten Vorzugsbereiche gelten dabei in gleicher Weise.

Die erfindungsgemäßen Verbindungen der Formel (I) sowie die erfindungsgemäßen Mischungen eignen sich insbesondere zur Herstellung von Fluorverbindungen aus den entsprechenden Hydroxyverbindungen sowie zur Herstellung von geminalen-Difluorverbindungen aus den entsprechenden Carbonylverbindungen.

Daher ist von der Erfindung auch ein Verfahren zur Herstellung von fluorhaltigen Verbindungen umfasst, das dadurch gekennzeichnet ist, dass Verbindungen enthaltend Hydroxy- und/oder Carbonylgruppen mit Verbindungen der Formel (I) und/oder den erfindungsgemäßen Mischungen umgesetzt werden.

Bevorzugte Verbindungen enthaltend Hydroxy- und/oder Carbonylgruppen sind solche, die zumindest eine aliphatische Hydroxygruppe und/oder zumindest eine Ketogruppe und/oder zumindest eine Aldehydgruppe und/oder eine Carboxylgruppe enthalten.

Besonders bevorzugte Verbindungen enthaltend Hydroxy- und/oder Carbonylgruppen sind solche, die eine aliphatische Hydroxygruppe oder eine Ketogruppe oder eine Aldehydgruppe oder eine Carboxylgruppe enthalten.

Die erfindungsgemäß herstellbaren fluorhaltigen Verbindungen eignen sich insbesondere zur Herstellung von Arzneimitteln, Agrochemikalien und Flüssigkristallen.

Die erfindungsgemäßen Verbindungen und Mischungen besitzen den Vorteil, dass sie einfach herzustellen und lagerstabil sind und die Umsetzung von Hydroxy- und Carbonylverbindungen zu den entsprechenden Fluor- und/oder Difluorverbindungen in hohen Ausbeuten ermöglichen. Die erfindungsgemäßen Verfahren zur Herstellung der oben genannten Verbindungen und Mischungen gehen von leicht verfügbaren Edukten aus und liefern die Produkte in hohen Ausbeuten.

**Beispiele:**

**Beispiel 1**

**Herstellung von 1,1-Dichlor-N,N-2,2-tetramethyl-1-propanamin**

27.8 g (210 mmol) N,N-Dimethylpivalamid und 250 ml tert. Butyl-methylether werden bei 20°C unter Schutzgas-Atmosphäre in einem Dreihalskolben mit KPG-Rührer vorgelegt. Zu dieser Reaktionsmischung tropft man 27.7 g (220 mmol) Oxalylchlorid, wobei sich während der Zugabe ein farbloser Feststoff abscheidet. Nach beendeter Zugabe rührt man weiter bis Ende der Gasentwicklung (ca. 2 h) und erwärmt den Ansatz zur Vervollständigung der Reaktion 0.5 h auf 40°C. Nach Entfernen aller flüchtigen Bestandteile im Hochvakuum erhält man 1,1-Dichlor-N,N-2,2-tetramethyl-1-propanamin als farblosen, hydrolyseempfindlichen Feststoff.

Ausbeute: 38.0 g (207 mmol; 96 %)

$^1$H-NMR ($CDCl_3$): 0.99 (s breit, 9H, *t*-Bu-*H*)*,* 3.46 (s, 6H, N(C*H*$_3$)$_2$).
$^{13}$C-NMR ($CDCl_3$): 28.1 ($CH_3$, 3C, *t*-Bu-$CH_3$), 29,5 (quart. C, 1C, *t*-Bu-C), 44,8 ($CH_3$, 1C, $NCH_3$), 51.1 ($CH_3$, 1C, $NCH_3$), 186.6 (quart. C, 1C, C-Cl) ppm.

**Beispiel 2**

**Herstellung von N,N-Diethyl-α,α-dichlor-3-pyridylmethanamin**

18.5 g (104 mmol) N,N-Diethylnicotinsäureamid und 150 ml tert. Butyl-methylether werden bei 20°C unter Schutzgas-Atmosphäre in einem Dreihalskolben mit KPG-Rührer vorgelegt. Zu dieser Reaktionsmischung tropft man 13.5 g (106 mmol) Oxalylchlorid, wobei sich während der Zugabe ein farbloser Feststoff abscheidet. Nach beendeter Zugabe rührt man 1 h bei 20°C und erhitzt den Ansatz zur Vervollständigung der Reaktion auf Rückfluß für weitere 4 h. Nach Abkühlen auf 20°C wird das Lösungsmittel im Wasserstrahlvakuum abgezogen, der verbleibende Rückstand wird mit wenig kaltem $Et_2O$ nachgewaschen. Nach Trocknen im Hochvakuum erhält man N,N-Diethyl-α,α-dichlor-3-pyridylmethanamin als leicht gelben Feststoff (Schmp.: 113-115°C).

Ausbeute: 22.9 g (98.8 mmol; 95%)

$^{1}H$-NMR ($CDCl_3$): 1.16 (s, 6H, $-CH_3$), 3.90 (s, 4H, $-CH_2$), 7.21 (s, 1H, arom.-H), 8.37 (s, 2H, arom.-H), 8.91 (s, 1H, arom.-H) ppm $^{13}C$-NMR ($CDCl_3$): 12.7 ($CH_3$, 2C, $-CH_3$), 55.6 ($-CH_2$, 2C, $NCH_2$-), 124.8 (-CH, 1C, arom.-C), 129.3 (-CH, 1C, arom.-C), 138.5 (-CH, 1C, arom.-C), 147.5 (-CH, 1C, arom.-C), 153.3 (-quart. C, 1C, arom.-C), 171.7 (quart. C, 1C, C-Cl) ppm.

Analog zu Beispiel 1 und 2 wurden hergestellt: 1,1-Dichlormethyl-N,N-dimethylamin (Beispiel 3), 1,1-Dichlormethyl-N,N-diethylamin (Beispiel 4), 1,1-Dichlormethyl-N,N-diisopropylamin (Beispiel 5), 1,1-Dichlor-N,N-2-trimethyl-1-propanamin (Beispiel 6), N,N-Diethyl-α,α-dichlor-2,2-dimethyl-1-propanamin (Beispiel 7), N-(1,1-Dichlor-methyl)-morpholin (Beispiel 8), 1,1-Dichlor-N,N-dimethyl-(p-chlorphenyl)Methanamin (Beispiel 9), 1,1-Dichlor-N,N-diisopropyl-phenylmethanamin (Beispiel 10), N,N-Dimethyl-α,α-dichlor-2-pyridylmethanamin (Beispiel 11) und 2,2-Dichlor-1,3,3-trimethylpyrrolidin (Beispiel 12).

Die Ausbeuten der Beispiele 3-12 sind in Tabelle 1 aufgeführt:

__Tabelle 1__

| Beispiel | Formel | Ausbeute | Beispiel | Formel | Ausbeute |
|---|---|---|---|---|---|
| 3 | 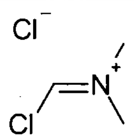 | 98 % | 8 | 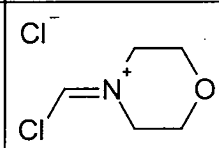 | 96 % |
| 4 | 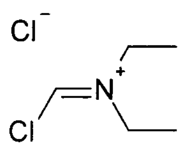 | 100 % | 9 | 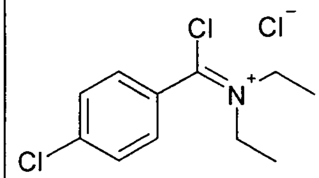 | 100 % |
| 5 | 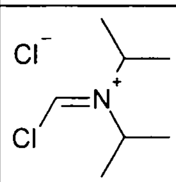 | 100 % | 10 | 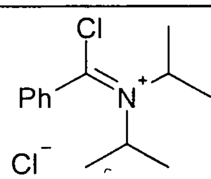 | 95 % |
| 6 | 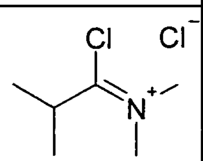 | 95 % | 11 | 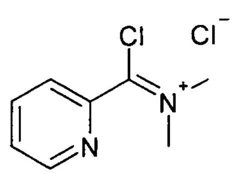 | 91 % |

| Beispiel | Formel | Ausbeute | Beispiel | Formel | Ausbeute |
|---|---|---|---|---|---|
| 7 | 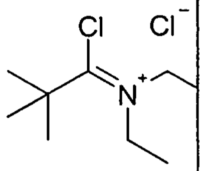 | 100 % | 12 | 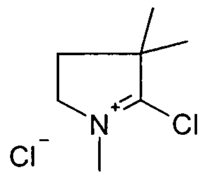 | 97 % |

**Beispiel 1a**

**Herstellung von 1,1-Difluor-N,N-2,2-tetramethyl-1-propanamin 1a**

Unter Schutzgas-Atmosphäre werden zu einer Suspension von 19,5 g (107 mmol) 1,1-Dichlor-N,N-2,2-tetramethyl-1-propanamin aus Beispiel 1 in 75 ml Dimethylimidazolidinon 17,8 g (424 mmol) Natriumfluorid gegeben und 25 h bei 20°C gerührt. Die anorganischen Salze werden unter Schutzgas-Atmosphäre abfiltriert und zweimal mit je 20 ml Dimethylimidazolidinon nachgewaschen. Das Rohprodukt wird im Hochvakuum aus der Reaktionslösung in eine auf -78°C gekühlte Vorlage überkondensiert und liefert nach anschließender fraktionierter Destillation im Vakuum (Sdp.: 62°C / 55 mbar) 1,1-Difluor-N,N-2,2-tetramethyl-1-propanamin als leicht gelben Flüssigkeit.

Ausbeute: 13.6 g (90 mmol; 84%)

$^{1}$H-NMR ($CDCl_3$): 1.00 (s breit, 9H, *t*-Bu-*H*), 2.26 (t, 6H, $^{4}J_{HF}$ = 1.95 Hz, $N(CH_3)_2$) ppm.
$^{13}$C-NMR ($C_6D_6$): 25.7 (s, $CH_3$, 3C, *t*-Bu-$CH_3$), 38.3 (t, $CH_3$, $^{3}J_{CF}$ = 6.03 Hz, $N(CH_3)_2$), 40.0 (t, quart. C, 1C, $^{2}J_{CF}$ = 29.8 Hz, *t*-Bu-C), 128.6 (t, $CF_2$, 1C, $^{1}J_{CF}$ = 258.1 Hz) ppm.
$^{19}$F-NMR ($CDCl_3$): -97.5 (s, $-CF_2$) ppm.
Analog dazu wurden die Beispiele 2a bis 12a durchgeführt. Die Parameter und Ausbeuten sind in Tabelle 2 wiedergegeben.

Tabelle 2

| Beispiel | Verbindung | Zeit [h] | Temp. [°C] | Solvens | Ausbeute [%] | Sdp. |
|---|---|---|---|---|---|---|
| 1a | 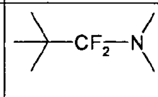 | 20 | 20 | $CH_3CN$ | 84 | 62°C 55 Torr |
| 2a | 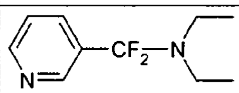 | 12 | 65-75 | $CH_3CN$ | 74 | 55°C 0.05 Torr |
| 3a | 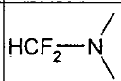 | 14-16 | 20 | DMF | 75 | 55°C |
| 4a | 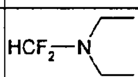 | 24 | 20 | $Et_2NCHO$ | 63 | 41°C 105 mm Hg |
| 5a | 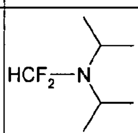 | 20 | 20 | $(i\text{-Pr})_2NCHO$ | 77 | 60°C 65 Torr |
| 6a | 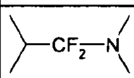 | 18-20 | 20 | $CH_3CN$ | 61 | 42°C 48 Torr |
| 7a | 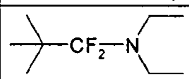 | 20 | 80 | $CH_3CN$ | 71 | 64°C 35 Torr |
| 8a | 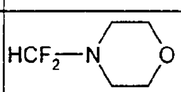 | 24 | 20 | DMI | 89 | --- |
| 9a | 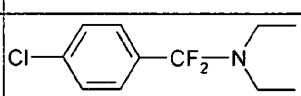 | 24 | 70 | $CH_3CN$ | 71 | --- |

| Beispiel | Verbindung | Zeit [h] | Temp. [°C] | Solvens | Ausbeute [%] | Sdp. |
|---|---|---|---|---|---|---|
| 10a | 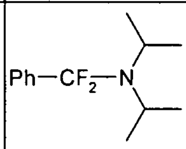 | 18 | 80 | $CH_3CN$ | 79 | --- |
| 11a | 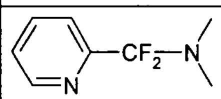 | 20 | 20 | DMI /$Me_4N$+F- | 95 | --- |
| 12a | 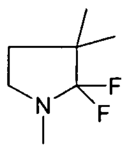 | 24 | 40 | DMI | 80.1 | 70°C<br>25 Torr |

<u>**Beispiel 3b**</u>

**Herstellung von Fluorierungsreagenzien enthaltend 1,1-Difluormethyl-N,N-dimethylamin (3b)**

Unter Schutzgas-Atmosphäre werden in einem Hochdruckbehälter 10 g (64 mmol) 1,1-Dichlormethyl-N,N-dimethylamin vorgelegt und auf 0°C abgekühlt. Dann werden 5.6 ml (320 mmol) HF zudosiert und der Ansatz unter Kühlung für 3 h gerührt. Nach beendeter Reaktion wird der Überschuss an HF und gebildetes HCl im Hochvakuum abgezogen. Man fügt dem Reaktionsgemisch 8.9 ml (64 mmol) Triethylamin hinzu und erhält 17.8 g (64 mmol) einer Mischung enthaltend **$Et_2N{=}CHF^+HF_2^-$ • $HNEt_3^+$• $HF_2^-$ (3b)** als eine leicht gelbe Flüssigkeit.

$^{19}F$-NMR ($CD_2Cl_2$): -89.2 (br s, 1F, $CHF^+$), -167.7 (br s, 4F, $HF_2^-$) ppm.

<u>**Beispiel 5b**</u>

**Herstellung von Fluorierungsreagenzien enthaltend 1,1-Difluormethyl-N,N-diisopropylamin (5b)**

Unter Schutzgas-Atmosphäre werden in einem Polyethylen-Kolben 10.4 g (68.9 mmol) 1,1-Difluormethyl-N, N-diisopropylamin vorgelegt und auf 0°C abgekühlt. Dann werden innerhalb von 2 min 11.1 g (68.9 mmol) $NEt_3$•3HF zudosiert und rührt noch 20 min bei dieser Temperatur. Die anfangs flüssig-kristalline Reaktionsmischung lässt man auf 20°C kommen, erwärmt zur Homogenisierung für 0.5 h auf 40°C und lässt wieder auf 20°C erkalten. Hieraus resultieren 21.5 g (68.9 mmol) ***i*-$Prop_2N{=}CHF^+HF_2^-$• $HNEt_3^+$• $HF_2^-$ (5b)** mit einem Schmelzpunkt von 37-40°C. $^{19}F$-NMR ($CD_2Cl_2$): -86.7 (br s, 1F, $CHF^+$), -158.5 (br s, 4F, $HF_2^-$) ppm.

<u>**Umsetzungen von Alkoholen mit α,α-Difluoraminen**</u>

<u>**Beispiel 13**</u> **(zum Vergleich)**

Man legt 6.8 g (50 mmol) 2,2-Difluor-1,3-dimethylimidazolidin unter Schutzgas-Atmosphäre vor und tropft hierzu eine Lösung von 5.5 g (45 mmol) 1-Phenylethanol in 20 ml $CH_3CN$. Der Reaktionsansatz wird für 6 h bei 20°C gerührt. Nach Reaktionsende versetzt man mit 30 ml 3 %iger $Na_2CO_3$-Lösung und extrahiert 3 mal mit je 50 ml n-Pentan. Nach Trocknen der vereinten organischen Phasen über $Na_2SO_4$ werden die flüchtigen Bestandteile abgezogen. Der Rückstand wird anschließend destilliert und liefert 2.9 g (23 mmol; 51 %) 1-Fluorethylbenzol (Sdp.: 52°C / 20 mbar).

$^{1}$H-NMR ($CDCl_3$): 1.60 (dd, 3H, $^{3}J_{HH}$ = 6.5 Hz, $^{3}J_{HF}$ = 24.1 Hz, -C$H_3$), 5.57 (dq, 1H, $^{3}J_{HH}$ = 6.5 Hz, $^{2}J_{HF}$ = 47.8 Hz, -*CH*F), 7.18-7.43 (m, 5H, arom-H) ppm.

$^{19}$F-NMR ($CDCl_3$): -168.2 (dq, 1F, $^{2}J_{HF}$ = 47.8 Hz, $^{3}J_{HF}$= 24.0 Hz, -CHF) ppm.

GC-MS: 124 [$M^+$], 109 [$M^+$-$CH_3$]

## Beispiel 14

Eine Lösung aus 9.51 g (63 mmol) 1,1-Difluormethyl-N,N-diisopropylamin **(5a)** wird unter Schutzgas-Atmosphäre vorgelegt. Zu dieser gerührten Lösung tropft man eine Lösung von 7.32 g (60 mmol) 1-Phenylethanol in 30 ml $CHCl_3$, erhitzt auf 60°C und rührt den Ansatz für 6 h. Nach Reaktionsende kühlt man auf 20°C ab, fügt 100 ml Eiswasser hinzu und extrahiert die wässrige Phase zweimal mit je 50 ml $CHCl_3$. Die vereinten organischen Phasen werden über $Na_2SO_4$ getrocknet, abfiltriert und einkonzentriert. Der Rückstand wird anschließend destilliert und liefert 6.45 g (52 mmol; 87%) 1-Fluorethylbenzol (Sdp.: 52°C / 20 mbar).

## Umsetzung von Alkoholen mit Fluorierungsreagenzien enthaltend α,α-Difluoramine

## Beispiel 15

In einem PE-Gefäß tropft man unter Schutzgas-Atmosphäre zu einer Lösung aus 2.32 g (7.56 mmol) ***i*-$Prop_2N{=}CHF^+HF_2^-$ • $HNEt_3^+$ • $HF_2^-$ (5b)** in 10 ml $CH_2Cl_2$ 0.83 g (6.8 mmol) 1-Phenylethanol innerhalb von 5 min. Man rührt für mehrere Stunden bei 20°C und analysiert den Umsatz $^{19}$F-NMR (Referenz: $PhCF_3$). Nach 2.5 h erhält man 81 % 1-Fluorethylbenzol, nach 24 h Rührzeit erhält man 96% Produkt.

## Beispiel 16

In einem PE-Gefäß legt man unter Schutzgas-Atmosphäre eine Lösung aus 12.4 g (44.4 mmol) **$Et_2N{=}CHF^+HF_2^-$ • $HNEt_3$ • $HF_2^-$ (3b)** vor und tropft hierzu 9.88 g (93.2 mmol) Benzaldehyd innerhalb von 10 min. Man rührt mehrere Stunden bei 80°C und analysiert den Umsatz mittels $^{19}$F-NMR (Referenz: $PhCF_3$). Nach 5 h Rührzeit erhält man 85 % Produkt.

## Beispiel 17

Eine Lösung aus 7,05 g (33 mmol) 1,1-Difluormethyl-N,N-diisopropylamin in 25 ml $CH_2Cl_2$ wird bei -15°C unter Schutzgas-Atmosphäre vorgelegt. Zu dieser gerührten Lösung tropft man eine Lösung von 10,0 g (31 mmol) N-tert.-Butoxycarbonyl-*trans*-4-hydroxy-*L*-prolin-benzylester in 25 ml $CH_2Cl_2$, lässt auf Raumtemperatur kommen und erhitzt unter Rühren für 3,5 h auf Rückfluss. Nach Reaktionsende kühlt man auf 20°C ab, fügt halbgesättigte $NaHCO_3$-Lösung hinzu und extrahiert die wässrige Phase zweimal mit je 50 ml $CH_2Cl_2$. Die vereinigten organischen Phasen werden über $Na_2SO_4$ getrocknet, abfiltriert und einkonzentriert. Der Rückstand wird anschließend destilliert und liefert 6,15 g (19 mmol; 61 %) N-tert.-Butoxycarbonyl-*trans*-4-fluor-*L*-prolinbenzylester.

## Umsetzungen mit α,α-Difluoraminen

Weitere Umsetzungen von Alkoholen mit 1,1-Difluor-N,N,2,2-tetramethyl-1-propanamin (1a) sind in Tabelle 3 wiedergegeben.

Tabelle 3

| | Substrat | Temp. [°C] | Zeit [h] | Solvens | Produkt | Ausbeute [%] |
|---|---|---|---|---|---|---|
| 18 | 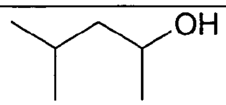 | 0 | 72 | $CH_2Cl_2$ | 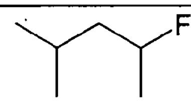 | 57 |
| 19 | 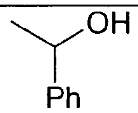 | 60 | 2 | $CHCl_3$ | 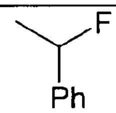 | 75 |
| 20 | 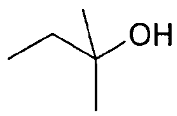 | 0 | 3 | $CH_2Cl_2$ | 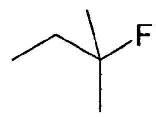 | 45 |
| 21 | n-$C_7H_{15}$—OH | 60 | 2 | $CHCl_3$ | n-$C_7H_{15}$—F | 57 |
| 22 | 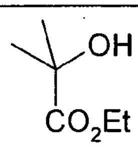 | 60 | 6 | $CHCl_3$ | 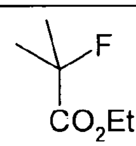 | 61 |
| 23 | 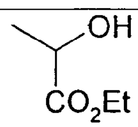 | 100 | 0.2 | Toluol | 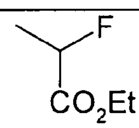 | 81 |

Umsetzungen von Alkoholen und Aldehyden mit 1,1-Difluormethyl-N,N-diethylamin (4a) sind in Tabelle 4 wiedergegeben.

**Tabelle 4**

| | Substrat | Temp.[ °C] | Zeit [h] | Solvens | Produkt | Ausbeute [%] |
|---|---|---|---|---|---|---|
| **23** | 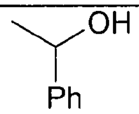 | 20 | 14 | $CH_2Cl_2$ | 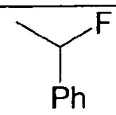 | 75 |
| **24** | 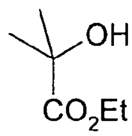 | 60 | 6 | $CHCl_3$ | 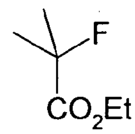 | 67 |
| **25** | 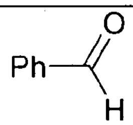 | 85 | 4 | - | 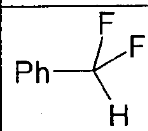 | 35* |

*Umsetzung mit 2 eq. α,α-Difluoramin

Umsetzungen von Alkoholen mit 1,1-Difluoromethyl-N,N-diisopropylamin (5a) sind in Tabelle 5 wiedergegeben.

**Tabelle 5**

| | Substrat | Temp.[ °C] | Zeit [h] | Solvens | Produkt | Ausbeute [%] |
|---|---|---|---|---|---|---|
| **26** | 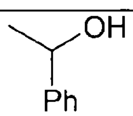 | 60 | 1 | $CHCl_3$ | 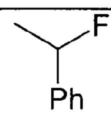 | 87 |
| **27** | 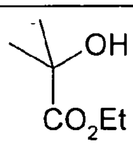 | 20 | 24 | $CHCl_3$ | 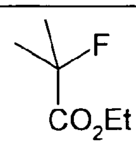 | 51 |
| **28** | 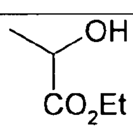 | 100 | 0.2 | Toluol | 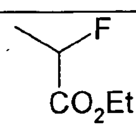 | 81 |

Umsetzungen von Alkoholen mit 1,1-Difluor-N,N-dimethylphenylmethanamin (10a) (als Vergleich) sind in Tabelle 6 wiedergegeben.

**Tabelle 6**

| | Substrat | Temp.[°C] | Zeit [h] | Solvens | Produkt | Ausbeute [%] |
|---|---|---|---|---|---|---|
| 29 | $n\text{-}C_7H_{15}\text{—}OH$ | 20 | 12 | $CH_3CN_3$ | $n\text{-}C_7H_{15}\text{—}F$ | 18 |
| 30 | 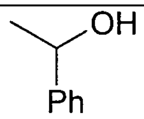 | 20 | 3 | $CHCl_3$ | 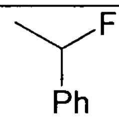 | 51 |
| 31 | 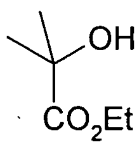 | 60 | 1.5 | $CHCl_3$ | 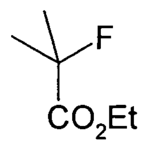 | 45 |
| 32 | 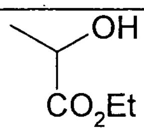 | 75 | 1.5 | $CHCl_3$ | 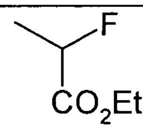 | 40 |
| 33 | 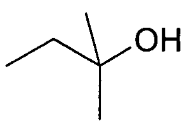 | 0 | 24 | $CHCl_3$ | 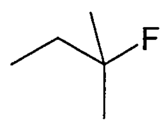 | 25 |
| 34 | 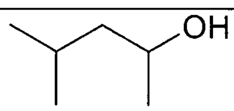 | 0 | 72 | $CHCl_3$ | 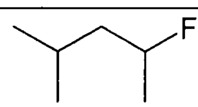 | 30 |

* Zugabe des α,α-Difluoramins

Umsetzungen von Alkoholen mit N,N-Diethyl-α,α-difluor-3-pyridinmethanamin (2a) sind in Tabelle 7 wiedergegeben:

Tabelle 7

| | Substrat | Temp.[ °C] | Zeit [h] | Solvens | Produkt | Ausbeute [%] |
|---|---|---|---|---|---|---|
| 35 | 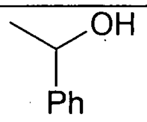 | 20 | 12 | $CH_2Cl_2$ | 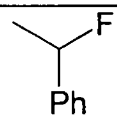 | 73 |
| 36 | 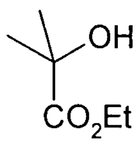 | 40 | 16 | $CHCl_3$ | 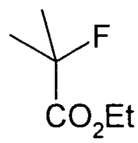 | 81 |
| 37 | 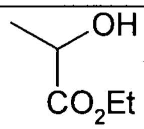 | 60 | 2 | $CHCl_3$ | 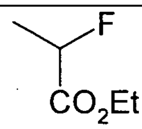 | 60 |

Umsetzungen von Alkoholen mit N,N-Dimethyl-α,α-difluor-2-pyridinmethanamin (11a) sind in Tabelle 8 wiedergegeben:

**Tabelle 8**

| | Substrat | Temp.[°C] | Zeit [h] | Solvens | Produkt | Ausbeute [%] |
|---|---|---|---|---|---|---|
| **38** | 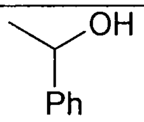 | 20 | 12 | $CH_2Cl_2$ | 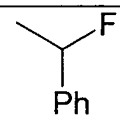 | 60 |
| **39** | 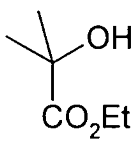 | 20 | 12 | $CH_2Cl_2$ | 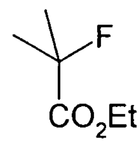 | 71 |
| **40** | 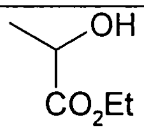 | 60 | 2 | $CHCl_3$ | 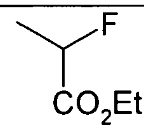 | 65 |

Umsetzungen von Alkoholen mit 2,2-Difluor-1,3,3-trimethylpyrrolidin (12a) sind in Tabelle 9 wiedergegeben:

**Tabelle 9**

| | Substrat | Temp.[°C] | Zeit [h] | Solvens | Produkt | Ausbeute [%] |
|---|---|---|---|---|---|---|
| **41** | 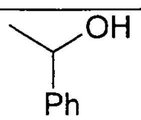 | 20 | 12 | $CHCl_3$ | 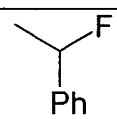 | 80 |

**Umsetzung mit Fluorierungsreagenzien enthaltend α,α-Difluoramine**

Umsetzungen von Alkoholen mit *i*-$Prop_2N{=}CHF^+HF_2^-\bullet HNEt_3^+\bullet HF_2^-$ (5b) sind in Tabelle 10 wiedergegeben:

**Tabelle 10**

| | Substrat | Temp.[ °C] | Zeit [h] | Solvens | Produkt | Ausbeute [%] |
|---|---|---|---|---|---|---|
| 42 | 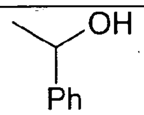 | 20 | 24 | $CH_2Cl_2$ | 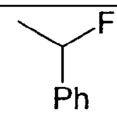 | 96* |

* laut $^{19}$F-NMR

Umsetzungen von Aldehyden mit 2eq $Et_2N{=}CHF^+HF_2^-$ • $HNEt_3$ • $HF_2^-$ (3b) sind in Tabelle 11 wiedergegeben:

**Tabelle 11**

| | Substrat | Temp.[ °C] | Zeit [h] | Solvens | Produkt | Ausbeute [%] |
|---|---|---|---|---|---|---|
| 43 | 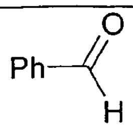 | 80-85 | 4-5 | --- | 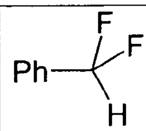 | 85* |

* laut $^{19}$F-NMR

**Patentansprüche**

1. Verbindungen der Formel (I)

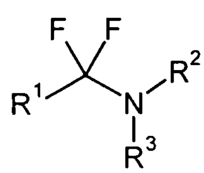 (I)

in der

| | |
|---|---|
| $R^1$ | für Wasserstoff, $C_1$-$C_{12}$-Alkyl, $[(C_2$-$C_{12}$-Alkylen)-O$]_n(C_1$-$C_{12}$-alkyl)] mit n = 1 bis 5, $C_4$-$C_{15}$-Arylalkyl oder $C_3$-$C_{14}$-Heteroaryl steht |
| $R^2$ und $R^3$ | jeweils unabhängig voneinander für $C_4$-$C_{15}$-Arylalkyl oder $C_1$-$C_{12}$-Alkyl stehen oder gemeinsam Teil eines cyclischen Restes mit insgesamt 3 bis 12 Kohlenstoffatomen sind oder |
| $R^1$ und $R^2$ und/oder $R^3$ | zusammen Teil eines cyclischen Restes mit insgesamt 3 bis 12 Kohlenstoffatomen sind, |

wobei 1,1-Difluormethyl-N,N-dimethylamin, 1,1-Difluormethyl-N,N-diethylamin, 1,1-Difluormethyl-N,N-diisopropylamin und 1,1-Difluor-N,N-2-trimethyl-1-propanamin ausgenommen sind.

2. Verbindungen nach Anspruch 1, **dadurch gekennzeichnet, dass** $R^1$ für Wasserstoff, $C_1$-$C_{12}$-Alkyl, oder $C_3$-$C_5$-Heteroaryl steht.

3. Verbindungen nach mindestens einem der Ansprüche 1 und 2, **dadurch gekennzeichnet, dass** $R^2$ und $R^3$ jeweils unabhängig voneinander für $C_1$-$C_8$-Alkyl stehen oder $NR^2R^3$ als Ganzes für N-Morpholinyl, N-Methyl-1,4-Piperazin-N-yl steht oder die Formel (I) als Ganzes für 2,2-Difluorpyrrolidin, 2,2-Difluorpiperidin, [2.2.2]-2,2,5,5-Tetrafluor-1,4-diazabicyclooctan oder [2.2.2]-2,2,6,6-Tetrafluor-1,4-diazabicyclooctan steht, wobei die genannten Reste gegebenenfalls einfach oder mehrfach durch $C_1$-$C_4$-Alkyl substituiert sind.

4. 1,1-Difluor-N,N-2,2-tetramethyl-1-propanamin, N,N-Diethyl-α,α-difluor-2,2-dimethyl-1-propanamin, N-(1,1-Difluormethyl)-morpholin, N,N-Diethyl-α,α-difluor-3-pyridylmethanamin, N,N-Diethyl-α,α-difluor-2-pyridylmethanamin und 2,2-Difluor-1,3,3-trimethylpyrrolidin.

5. Mischungen enthaltend

- Verbindungen der Formel (Ia)

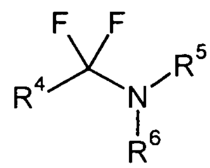

(Ia)

in der

| | |
|---|---|
| $R^4$ | für Wasserstoff, $C_1$-$C_{12}$-Alkyl, $[(C_2$-$C_{12}$-Alkylen)-O$]_n$($C_1$-$C_{12}$-alkyl)] mit n = 1 bis 5, $C_3$-$C_{14}$-Aryl oder $NR^7R^8$ steht, wobei $R^7$ und $R^8$ jeweils unabhängig voneinander für $C_1$-$C_8$-Alkyl stehen oder $NR^7R^8$ als Ganzes für einen 4- bis 7-gliedrigen cyclischen Rest mit insgesamt 3 bis 12 Kohlenstoffatomen steht und |
| $R^5$ und $R^6$ | jeweils unabhängig voneinander für $C_1$-$C_{12}$-Alkyl stehen oder gemeinsam Teil eines cyclischen Restes mit insgesamt 4 bis 12 Kohlenstoffatomen sind oder |
| $R^4$ und $R^5$ und/oder $R^6$ | zusammen Teil eines cyclischen Restes mit insgesamt 4 bis 12 Kohlenstoffatomen sind, |

- zumindest ein aprotisches, tertiäres Amin das in α-Stellung zum Stickstoff keine Fluoratome enthält und/oder zumindest eine N-heteroaromatische Verbindung und

- Fluorwasserstoff.

6. Mischungen nach Anspruch 5, **dadurch gekennzeichnet, dass** das molare Verhältnis von aprotischem tertiärem Amin und/oder N-heteroaromatischer Verbindung zu Verbindungen der Formel (Ia) 0,1:1 bis 20:1 beträgt.

7. Mischungen nach mindestens einem der Ansprüche 5 und 6, **dadurch gekennzeichnet, dass** das molare Verhältnis von Fluorwasserstoff zu aprotischem tertiärem Amin und/oder N-heteroaromatischer Verbindung 0,2: 1 bis 10:1 pro Stickstoffatom beträgt.

8. Mischungen nach mindestens einem der Ansprüche 5 bis 7, **dadurch gekennzeichnet, dass** als Verbindungen der Formel (Ia) solche der Formel (I) mit der in Anspruch 1 genannten Bedeutung eingesetzt werden oder solche der Formeln (Ib), (Ic), (Id) oder (Ie)

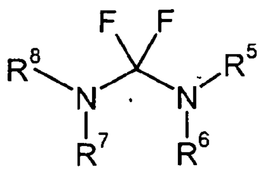
(Ib)

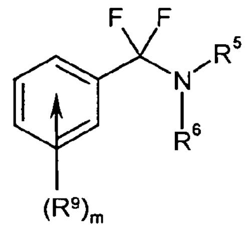
(Ic)

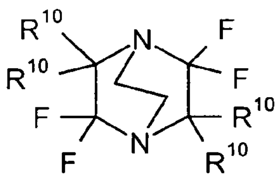
(Id)

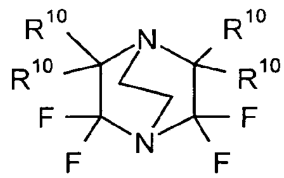
(Ie)

in denen

$R^5$, $R^6$, $R^7$ und $R^8$ die in Anspruch 5 genannte Bedeutung besitzen,

m für 0, 1, 2, 3 oder 4 steht und

$R^9$ für Reste steht, die ausgewählt sind aus der Gruppe Chlor, Fluor, $C_1$-$C_{12}$-Alkyl, $C_1$-$C_{12}$-Fluoralkyl, $C_1$-$C_{12}$-Fluoralkoxy, $C_1$-$C_{12}$-Fluoralkylthio, $C_1$-$C_{12}$-Alkcoxy und Di($C_1$-$C_8$-alkyl)-amino und

$R^{10}$ jeweils unabhängig für Wasserstoff oder $C_1$-$C_{12}$-Alkyl steht.

**9.** Verfahren zur Herstellung von Verbindungen gemäß mindestens einem der Ansprüche 1 bis 4, **dadurch gekennzeichnet, dass** Verbindungen der Formel (V)

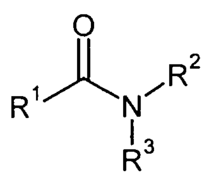
(V)

in der $R^1$, $R^2$ und $R^3$ die in Anspruch 1 angegebene Bedeutung besitzen

- in einem Schritt a) mit Halogenierungsmittel in Verbindungen der Formel (VI) überführt werden

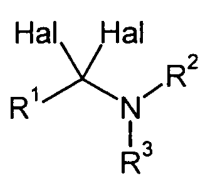
(VI)

in der Hal jeweils unabhängig für Chlor oder Brom steht und

- in einem Schritt b) die Verbindungen der Formel (VI) mit ionischem Fluorid in Verbindungen der Formel (I) überführt werden.

**10.** Verfahren nach Anspruch 9, **dadurch gekennzeichnet, dass** als Halogenierungsmittel für Schritt a) Phosphorpentachlorid, Phosphorpentabromid, Thionylchlorid, Thionylbromid, Phosgen und/oder Oxalsäurechlorid eingesetzt werden.

**11.** Verfahren nach mindestens einem der Ansprüche 9 und 10, **dadurch gekennzeichnet, dass** als ionische Fluoride quartäre Ammonium- oder Phosphoniumfluoride sowie Alkalimetallfluoride oder Mischungen der genannten Verbindungen eingesetzt werden.

**12.** Verfahren nach mindestens einem der Ansprüche 9 bis 11, **dadurch gekennzeichnet**, die Reaktivität der ionischen Fluoride durch Zusätze modifiziert wird.

**13.** Verfahren zur Herstellung von Mischungen gemäß mindestens einem der Ansprüche 5 bis 8, **dadurch gekennzeichnet, dass** Verbindungen der Formel (VI) mit der in Anspruch 9 genannten Bedeutung in Gegenwart von Fluorwasserstoff umgesetzt und gegebenenfalls die auf diese Weise erhaltene Reaktionsmischung mit aprotischem tertiärem Amin das in α-Stellung zum Stickstoff keine Fluoratome enthält und/oder N-heteroaromatischer Verbindung umgesetzt wird.

**14.** Verfahren nach Anspruch 13, **dadurch gekennzeichnet, dass** Verbindungen der Formel (VI) mit so viel Fluorwasserstoff umgesetzt werden und der so erhaltenen Reaktionsmischung so viel aprotisches, tertiäres Amin das in α-Stellung zum Stickstoff keine Fluoratome enthält und/oder N-heteroaromatischer Verbindung zugesetzt wird, dass das molare Verhältnis von aprotischem tertiärem Amin und/oder N-heteroaromatischer Verbindung zu Verbindungen der Formel (Ia) 0,1:1 bis 20:1 beträgt und das molare Verhältnis von Fluorwasserstoff zu aprotischem tertiärem Amin und/oder N-heteroaromatischer Verbindung 0,2:1 bis 10: 1 pro Stickstoffatom beträgt.

**15.** 1,1-Dichlormethyl-N,N-dimethylamin, 1,1-Dichlormethyl-N,N-diethylamin, 1,1-Dichlormethyl-N,N-diisopropylamin, 1,1-Dichlor-N,N-2-trimethyl-1-propanamin, 1,1-Dichlor-N,N-2,2-tetramethyl-1-propanamin, N,N-Diethyl-α,α-dichlor-2,2-dimethyl-1-propanamin, N-(1,1-Dichlormethyl)-morpholin, 1,1-Dichlor-N,N-dimethylphenylmethanamin, N,N-Diethyl-α,α-dichlor-3-pyridylmethanamin, N,N-Diethyl-α,α-dichlor-2-pyridylmethanamin und 2,2-Dichlor-1,3,3-trimethylpyrrolidin.

**16.** Verfahren zur Herstellung von fluorhaltigen Verbindungen, **dadurch gekennzeichnet, dass** Verbindungen enthaltend Hydroxy- und/oder Carbonylgruppen mit Verbindungen gemäß mindestens einem der Ansprüche 1 bis 4 und/oder Mischungen gemäß mindestens einem der Ansprüche 5 bis 8 umgesetzt werden.

**17.** Verfahren nach Anspruch 16, **dadurch gekennzeichnet, dass** als Verbindungen enthaltend Hydroxy- und/oder Carbonylgruppen solche eingesetzt werden, die zumindest eine aliphatische Hydroxygruppe und/oder zumindest eine Ketogruppe und/oder zumindest eine Aldehydgruppe und/oder eine Carboxylgruppe enthalten.

**18.** Verwendung von Verbindungen gemäß mindestens einem der Ansprüche 1 bis 4 oder Mischungen gemäß mindestens einem der Ansprüche 5 bis 8 zur Herstellung von Fluorverbindungen aus den entsprechenden Hydroxyverbindungen sowie zur Herstellung von geminalen-Difluorverbindungen aus den entsprechenden Carbonylverbindungen.

**19.** Verwendung von fluorhaltigen Verbindungen, die gemäß mindestens einem der Ansprüche 16 und 17 hergestellt wurden zur Herstellung von Arzneimitteln, Agrochemikalien oder Flüssigkristallen.